(19) Europäisches Patentamt
European Patent Office
Office européen des brevets

(11) Veröffentlichungsnummer: **0 313 113**
**A1**

(12) # EUROPÄISCHE PATENTANMELDUNG

(21) Anmeldenummer: **88201716.3**

(22) Anmeldetag: **11.08.88**

(51) Int. Cl.4: **C07C 59/10 , C08K 5/00 , C08L 57/08 , //(C08K5/00, 5:09,5:13),(C08K5/00,5:09,5:34), (C08L57/08,61:04)**

(30) Priorität: **23.10.87 DE 3735863**

(43) Veröffentlichungstag der Anmeldung: **26.04.89 Patentblatt 89/17**

(84) Benannte Vertragsstaaten: **BE DE FR GB IT SE**

(71) Anmelder: **METALLGESELLSCHAFT AG**
**Reuterweg 14 Postfach 3724**
**D-6000 Frankfurt/M.1(DE)**

(72) Erfinder: **Baum, Norbert**
**Nachtigallenstrasse 46**
**D-6078 Neu-Isenburg(DE)**
Erfinder: **Basista, Gabriele**
**Leerbachstrasse 106**
**D-6000 Frankfurt am Main(DE)**

(54) **3-basisches Bleidimethylolpropionat und dieses enthaltende Stabilisatorzusammensetzung für halogenhaltige Vinylpolymerisate.**

(57) Die Erfindung betrifft ein 3-bas. Bleidimethylolpropionat der Formel $3PbO \times Pb [CH_3-C(CH_2OH)_2COO]_2$ als neue Bleiverbindung sowie eine diese Verbindung enthaltende Stabilisatorzusammensetzung für halogenhaltige Vinylpolymerisate. Das 3-bas. Bleidimethylolpropionat wird hierin in Kombination mit einem Oxidationsmittel eingesetzt, sowie ggf. mit weiteren üblichen Stabilisatoren, Hilfs- und Füllstoffen.

EP 0 313 113 A1

# 3-basisches Bleidimethylolpropionat und dieses enthaltende Stabilisatorzusammensetzung für halogenhaltige Vinylpolymerisate

Die Erfindung betrifft dreibasisches Bleidimethylolpropionat sowie eine dieses enthaltende Stabilisatorzusammensetzung für halogenhaltige Vinylpolymerisate.

Die Stabilisierung halogenhaltiger Vinylpolymerisate mit basischen Bleisalzen organischer oder anorganischer Säuren bzw. mit Mischungen dieser Salze ist seit langem bekannt. Dabei ist es auch bekannt, weitere Hilfsstabilisatoren, Gleitmitel, Füll- und Hilfsstoffe, sowie Antioxidantien, den Zusammensetzungen einzuverleiben.

Unter basischen Bleisalzen seien z.B. zweibasisches Bleiphthalat, zweibasisches Bleiphosphit, basisches Bleicarbonat, basisches Bleisilikat und dreibasisches Bleisulfat erwähnt. Obwohl solche basischen Bleisalze sehr vorteilhaft als Stabilisierungsmittel verwendet werden können, zeigen sie trotzdem einige Nachteile. In einigen Fällen sind diese Produkte zu reaktionsfähig, indem sie mit organischen Farbstoffen oder mit Weichmachern reagieren, wobei sie unter bestimmten Bedingungen eine Verfärbung verursachen und die Weichmacher teilweise verseifen. Die Widerstandsfähigkeit gegen Feuchtigkeit einiger dieser basischen bleihaltigen Stabilisierungsmittel ist unter gewissen Bedingungen unzureichend, so daß die elektrischen Isoliereigenschaften solcher bleihaltigen Formmassen nach längerem Einfluß von Wasser stark abfallen.

Es ist des weiteren bekannt, daß für die Stabilisierung von insbesondere weich eingestellten halogenhaltigen Vinylpolymerisatmassen sich Bleiweiß und vierbasisches Bleifumarat als wirksamste basische Bleiverbindungen erwiesen haben.

Die Herstellung von basischen Bleisalzen organischer und anorganischer Säuren erfolgt in aller Regel auf naßchemischem Wege durch Umsetzung der betreffenden Säure mit Bleioxid in einer der Stöchiometrie der herzustellenden Verbindung entsprechenden Menge, zweckmäßig in Gegenwart eines Katalysators, wie Ameisen-oder Essigsäure, Alkohol oder ferner auch durch Umsetzung von Bleioxid in geschmolzener Fettsäure.

Die Dimethylolpropionsäure ist eine trifunktionelle Verbindung mit Neopentylstruktur mit zwei primären Hydroxylgruppen und einer tertiären Carboxylgruppe im Molekül und weist neben ihrer Trifunktionalität eine gewisse sterische Hinderung auf.

Es wurde nun gefunden, daß Dimethylolpropionsäure mit Bleioxid eine neue sehr stabile Verbindung in Form eines dreibasischen Bleisalzes bildet. Die Erfindung betrifft daher ein neues Bleisalz der Formel

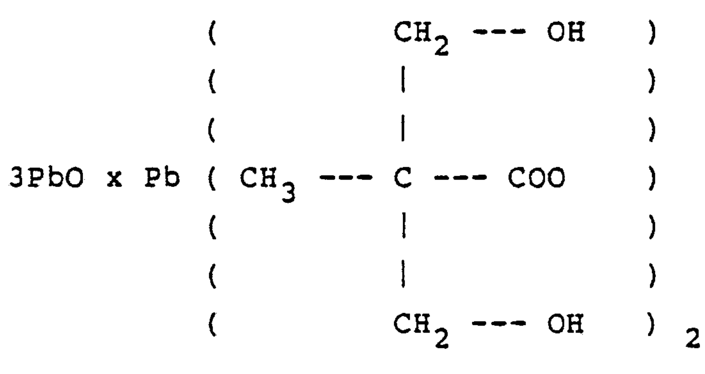

Das neue Bleisalz hat einen Bleigehalt von 73,3 % bei einem theoretischen Bleigehalt von 72,5 %. Der Glühverlust (2 h bei 500 °C) beträgt 25,7 %. Die Dichte des neuen Bleisalzes ist 4,5 g/cm$^3$.

Die Oberflächenmorphologie des neuen dreibasischen Bleidimethylolpropionats wurde mittels Rasterelektronenmikroskop bestimmt. Danach ist das dreibasische Bleidimethylolpropionat aus sehr feinen stäbchenförmigen Einzelteilchen mit Durchmessern von ca. 0,2 bis 0,5 µm und Längen von ca. 0,5 bis 10 µm aufgebaut, welche Feinheit eine gute Dispergierbarkeit im PVC gewährleistet.

Es wurde des weiteren gefunden, daß sich das dreibasische Bleidimethylolpropionat in hervorragender Weise als Stabilisator für halogenhaltige Vinylpolymerisate verwenden läßt. Beispielsweise ist die Stabilisierungswirkung (Wärmestabilität) von dreibasischem Bleidimethylolpropionat in einer Standardrezeptur bei Verwendung von 1 bis 5 Gew.-Teilen Stabilisator auf 100 Gew.-Teilen halogenhaltiges Vinylpolymerisat um etwa 20 % besser als bei Einsatz von dreibasischem Bleisulfat. Gegenstand der Erfindung ist daher ferner

eine Stabilisatorzusammensetzung für halogenhaltige Vinylpolymerisate, insbesondere weichmacherhaltige, halogenhaltige Vinylpolymerisate, auf Basis basischer Bleisalze sowie mit gegebenenfalls weiteren üblichen Stabilisatoren, Hilfs- und Füllstoffen, die dadurch gekennzeichnet ist, daß sie dreibasisches Bleidimethylolpropionat und ein Antioxydans enthält. Es hat sich ferner gezeigt, daß die gleichzeitige Anwesenheit eines Antioxydans in der Zusammensetzung für die volle Wirksamkeit des dreibasischen Bleidimethylolpropionats wesentlich ist. Besonders geeignet ist ein Antioxydans aus der Gruppe Bisphenol A, -Phenylindol, Kondensationsprodukt aus 3-Methyl-6-tert.butylphenol und Crotonaldehyd, wobei in einem bevorzugten Kondensationsprodukt die Komponenten im Molverhältnis von 3 : 1 kondensiert worden sind.

Es wurde des weiteren gefunden, daß das dreibasische Bleidimethylolpropionat in Verbindung bzw. Mischung mit üblichen, als Bleistabilisatoren verwendeten basischen Bleisalzen eine synergistische Wirkung herbeiführt und die Langzeitstabilität erhöht.

Bei der Stabilisierung halogenhaltiger Vinylpolymerisate üblicherweise verwendete neutrale oder basische Bleisalze sind beispielsweise neutrales oder 1- bis 2-basisches Bleistearat, Bleiweiß, 3- bis 4-basisches Bleisulfat, 2-basisches Bleiphosphit, 2-basisches Bleisulfat, 2-basisches Bleiphthalat, 4-basisches Bleifumarat.

Üblicherweise wird die Stabilisatorzusammensetzung in folgenden Mengenverhältnissen der Komponenten verwendet:

5 bis 99,5 Gew.-% dreibasisches Bleidimethylolpropionat
0 bis 87 Gew.-% herkömmliches basisches Bleisalz
0,5 bis 8 Gew.-% Antioxydans
0 bis 45 Gew.-% übliche Hilfs- und Füllstoffe

Die Stabilisatorzusammensetzung wird in einer Menge von 1 bis 10 Gew.-%, bezogen auf weichmacherhaltiges, halogenhaltiges Vinylpolymerisat, eingesetzt.

Übliche Hilfs- und Füllstoffe der Stabilisatorzusammensetzung sind beispielsweise Gleitmittel, Weichmacher, Hilfsstabilisatoren, Pigmente, Farbstoffe. Bevorzugte Weichmacher sind Di-2-äthylhexylphthalat, Di-iso-decylphthalat, Di-2-äthylhexyladipat, Di-n-octylsebacat, Tri-n-$C_{8-10}$-alkyltrimellitat, Di-n-$C_{10-12}$-alkylphthalat.

Das am häufigsten verwendete Vinylhalogenidpolymerisat ist ein Vinylchloridhomopolymerisat, d.h. Polyvinylchlorid. Die Erfindung ist jedoch nicht auf den Einsatz der Stabilisatorzusammensetzung in Polyvinylchlorid beschränkt, sondern andere halogenhaltige, erfindungsgemäß verwendbare Polymerisate umfassen chloriertes Polyäthylen, chloriertes Polyvinylchlorid und Vinylhalogenidpolymerisate. Bei den letzteren handelt es sich um Polymerisate, die durch Polymerisation oder Mischpolymerisation von Vinylmonomeren mit oder ohne andere Comonomere, wie Äthylen, Vinylacetat, Vinyläther, Vinylidenchlorid, Methacrylate, Acrylate, Styrol usw., erhalten werden. Weitere Vinylhalogenidpolymerisate sind beispielsweise Vinylidenchloridpolymerisate, Vinylchlorid/Vinylester-, Vinylchlorid/Vinyläther-, Vinylchlorid/Vinylidenchlorid, Vinylchlorid/Propylen-Copolymerisate, chloriertes Polyäthylen.

Die erfindungsgemäße Stabilisatorzusammensetzung wird insbesondere in Kabelrezepturen eingesetzt. Derartige Kabelrezepturen haben im allgemeinen eine Zusammensetzung von:

| | |
|---|---|
| PVC | 58,0 % |
| Weichmacher | 24,0 % |
| Füllstoff | 14,5 % |
| Stabilisator | 2,3 % |
| Gleitmittel | 0,7 % |
| Pigment | 0,5 % |

Die Vorteile der Stabilisatorzusammensetzung der Erfindung sind in der verbesserten Langzeitstabilisierung zu sehen, insbesondere, wenn eine hohe Wärmestabilität benötigt wird und die PVC-Mischung hohe Stabilisatorenanteile enthalten muß.

Die elektrischen Eigenschaften der mit dreibasischem Bleidimethylolpropionat stabilisierten Kabelmassen hinsichtlich des Oberflächen-, Durchgangs- und Isolationswiderstandes sind mit herkömmlichen Bleisalzen stabilisierten Kabelmassen vergleichbar.

Die Erfindung wird anhand der nachstehenden Beispiele näher und beispielhaft erläutert.

In eine Mischung für Kabelmassen der Zusammensetzung

100 Gew.-Teile Suspensions-PVC, K-Wert 70
50 Gew.-Teile Di-2-äthylhexylphthalat
0,5 Gew.-Teile n-Paraffin
0,5 Gew.-Teile Wachsester
0,3 Gew.-Teile Bisphenol A

wurden jeweils 1 bis 5 Gew.-Teile folgender basischer Bleisalze eingearbeitet:

dreibasisches Bleisulfat, 0,5-basisches Bleicarbonat, zweibasisches Bleiphthalat, vierbasisches Bleifumarat, dreibasisches Bleidimethylolpropionat.

Die Mischungen wurden homogenisiert und während 10 min. bei 170°C auf einem Walzwerk zu Folien einer Stärke von 0,5 mm gewalzt und der HCl-Wert nach VDE 0271 bestimmt. Dazu wurden aus den Folien Probestreifen der Abmessung 20 x 3 mm hergestellt. Sodann wurden jeweils 50 mg der Probestreifen in Glasröhrchen (Länge 115 mm, Durchmesser 5 mm, Wandstärke 0,5 mm) überführt. Anschließend wurden die Glasröhrchen mit Universalindikatorpapier versehen, in einen auf 200°C temperierten Metallblock-Thermostaten eingeführt und hierauf die Zeit bis zum Farbumschlag des Indikatorpapiers nach rötlich gemessen.

Der Tabelle 1 mit den Versuchs-Nummern 1 bis 20 ist zu entnehmen, daß das dreibasische Bleidimethylolpropionat eine höhere Stabilisierungswirkung zeigt als die herkömmlichen und für Kabelrezepturen in Weich-PVC bevorzugten herkömmlichen basischen Bleistabilisatoren.

Wie die Tabelle 1 zeigt, wurde nach den Versuchen 6 bis 10 der HCL-Wert (Dosierung 2,5 Gew.-Teile Bleistabilisatoren auf 100 Gew.-Teile Suspensions-PVC) für Massen mit den Stabilisatoren

dreibasisches Bleisulfat mit 74 min,
0,5-basisches Bleicarbonat mit 69 min,
zweibasisches Bleiphtalat mit 67 min,
vierbasisches Bleifumarat mit 79 min

bestimmt. Demgegenüber weist das erfindungsgemäß mit dreibasischem Bleidimethylolpropionat stabilisierte PVC einen HCl-Wert von 92 min auf.

In einer weiteren Versuchsreihe wurde die synergistische Wirkung des dreibasischen Bleidimethylolpropionats in Mischung mit herkömlichen basischen Bleisalzen untersucht. Es wurde wiederum die in den Versuchen 1 bis 20 beschriebene Mischung für Kabelmassen der Zusammensetzung

100 Gew.-Teile Suspensions-PVC, K-Wert 70
50 Gew.-Teile Di-2-äthylhexylphthalat
0,5 Gew.-Teile n-Paraffin
0,5 Gew.-Teile Wachsester
0,3 Gew.-Teile Bisphenol A

verwendet.

In diese Mischung wurden jeweils 1,25 und 2,5 Gew.-Teile dreibasisches Bleidimethylolpropionat zusammen mit je 1,25 und 2,5 Gew.-Teile folgender basischer Bleisalze eingearbeitet:

dreibasisches Bleisulfat, 0,5-basisches Bleicarbonat, zweibasisches Bleiphthalat, vierbasisches Bleifumarat.

Die Mischungen wurden, wie für die Versuche Nr. 1 bis 20 beschrieben, entsprechend weiterverarbeitet und die HCl-Werte bei 200°C nach VDE 0271 bestimmt.

Der Tabelle 2 mit den Versuchs-Beispielen 21 bis 28 ist zu entnehmen, daß die Abmischungen des dreibasischen Bleidimethylolpropionats mit herkömmlichen Bleisalzen im Gewichtsverhältnis 1 : 1 in einer Dosierung von 2,5 und 5 Gew.-Teile/100 Gew.-Teile Suspensions-PVC, im Vergleich zu den Einzelkomponenten der Versuche 6 bis 10 (TAB. 1a), eine höhere Stabilisierwirkung zeigen. So weist das 0,5-basische Bleicarbonat in einer Dosierung von 2,5 Gew.-Teile/100 Gew.-Teile S-PVC einen HCl-Wert von 69 min auf (Versuch 7). Das dreibasische Bleidimethylolpropionat weist in einer Dosierung von 2,5 Gew.-Teile/100 Gew.-Teile S-PVC einen HCl-Wert von 92 min auf (Versuch 10), während das erfindungsgemäße Gemisch von 1,25 Gew.-Teile 0,5-basisches Bleicarbonat und 1,25 Gew.-Teile dreibasisches Bleidimethylolpropionat jeweils /100 Gew.-Teile S-PVC einen höheren HCl-Wert von 99 min (Versuch 22) gegenüber den Einzelkompenenten aufweist.

In einer weiteren Versuchsreihe wurde die Stabilisierwirkung des dreibasischen Bleidimethylolpropionats

im Vergleich zu herkömmlichen basischen Bleisalzen untersucht, wobei jedoch gegenüber den Versuchen 1 bis 20 als Weichmacher das Di-iso-decylphthalat eingesetzt wurde.

In eine Mischung für Kabelmassen der Zusammensetzung

100 Gew.-Teile Suspensions-PVC, K-Wert 70
50 Gew.-Teile Di-iso-decylphthalat
0,5 Gew.-Teile n-Paraffin
0,5 Gew.-Teile Wachsester
0,3 Gew.-Teile Bisphenol A

wurden jeweils 2,5 und 5,0 Gew.-Teile folgender basischer Bleisalze eingearbeitet:

dreibasisches Bleisulfat, 0,5-basisches Bleicarbonat, zweibasisches Bleiphthalat, vierbasisches Bleifumarat, dreibasisches Bleidimethylolpropionat.

Die Mischungen wurden, wie für die Versuche Nr. 1 bis 20 beschrieben, entsprechend weiterverarbeitet und die HCl-Werte bei 200 °C nach VDE 0271 bestimmt.

Der Tabelle 3 mit den Versuchs-Beispielen 29 bis 38 ist zu entnehmen, daß das dreibasische Bleidimethylolpropionat eine höhere Stabilisierwirkung zeigt als die herkömmlichen und für Kabelrezepturen in Weich-PVC bevorzugten basischen Bleistabilisatoren.

Des weiteren wurde das dreibasische Bleidimethylolpropionat in Kabelmischungen mit hoher Thermostabilität untersucht, wie z.B. die Isoliermischung vom Typ "YI8" nach VDE 0207, Teil 4, oder die Mantelmischung "YM4" nach VDE 0207, Teil 5, mit einer höchst zulässigen Betriebstemperatur am Leiter von 90 °C.

In einer Mischung für Kabelmassen der Zusammensetzung

100 Gew.-Teile Suspensions-PVC, K-Wert 70
50 Gew.-Teile Tri-n-$C_8$-$C_{10}$-alkyltrimellitat
0,5 Gew.-Teile n-Paraffin
1,0 Gew.-Teile Wachsester
0,25 Gew.-Teile Bisphenol A

wurden jeweils 10, 12 und 13 Gew.-Teile zweibasisches Bleiphthalat, 8 und 10 Gew.-Teile vierbasisches Bleifumarat, 7, 8 und 10 Gew.-Teile dreibasisches Bleidimethylolpropionat eingearbeitet.

Die Mischungen wurden, wie für die Versuche Nr. 1 bis 20 beschrieben, entsprechend weiterverarbeitet und die HCl-Werte bei 200 °C nach VDE 0271 bestimmt.

Sodann wurden aus den Folien Probestreifen der Abmessung 5 cm x 3 cm hergestellt und auf einem Nesseltuch in einem Umluftwärmeschrank (Typ UT 5042 E - Fa. Heraeus) 14 Tage lang bei 140 °C gelagert. Anschließend wurde die Reststabilität des gelagerten Probestreifens bei 200 °C nach VDE 0271 bestimmt.

Der Tabelle 4 mit den Versuchs-Beispielen 39 bis 46 ist zu entnehmen, daß das dreibasische Bleidimethylolpropionat eine höhere Reststabilität zeigt gegenüber den für dieses Einsatzgebiet üblichen Bleisalzen.

Gemäß Tabelle 4 weisen das zweibasische Bleiphthalat in einer Dosierung von 10 Gew.-Teilen/100 Gew.-Teile S-PVC einen HCl-Wert nach 14-tägiger Lagerung von 75 min auf (Versuch 39) und das vierbasische Bleifumarat in einer Dosierung von 10 Gew.-Teile/100 Gew.-Teile S-PVC einen HCl-Wert nach 14-tägiger Lagerung von 171 min auf (Versuch 43). Demgegenüber weist das erfindungsgemäße dreibasische Bleidimethylolpropionat einen höheren HCl-Wert nach 14-tägiger Lagerung von 240 min auf (Versuch 46). Auch die entsprechenden nicht gelagerten Probestreifen zeigen, daß das dreibasische Bleidimethylolpropionat mit einem HCl-Wert von 570 min gegenüber den 355 min des zweibasischen Bleiphthalats und den 432 min des vierbasischen Bleifumarats ein hervorragender Wärmestabilisator ist.

Die nach VDE 0207 für eine "YI8" oder "YM4" Mischung vorgeschriebene Mindestreststabilität beträgt 120 min. Aus den Versuchen 40, 42 und 44 der Tabelle 4 geht hervor, daß diese Reststabilität unter den gegebenen Bedingungen mit 12 Gew.-Teile zweibasisches Bleiphthalat/100 Gew.-Teile S-PVC oder 8 Gew.-Teile vierbasisches Bleifumarat/100 Gew.-Teile S-PVC oder mit 7 Gew.-Teile dreibasisches Bleidimethylolpropionat/100 Gew.-Teile S-PVC erzielt wird.

Das erfindungsgemäße dreibasische Bleidimethylolpropionat weist somit aufgrund der höheren Stabilisierungswirksamkeit den erheblichen Vorteil auf, daß es gegenüber den herkömmlichen Bleisalzen bei gleicher Reststabilität niedriger dosiert werden kann.

Ferner wurde an den in den Versuchen Nr. 39 bis 46 hergestellten Mischungen der spezifische Durchgangswiderstand nach DIN 53 482, VDE 0303, bestimmt. Dazu wurden aus den Folien jeweils 6 Folienstücke von 0,5 mm Stärke und dem Gesamtgewicht von 50 g bei 170 °C zu 2 mm starken Platten verschweißt. Aus diesen Platten wurden anschließend kreisförmige Probekörper mit einer Fläche von 50 $cm^2$ hergestellt und mit einer Haftschicht von Leitsilber versehen. Danach wurde der spezifische Durchgangswiderstand bei einer Temperatur von 23 °C und der rel. Luftfeuchte von 56 % gemessen.

Aus der Tabelle 4 und den Versuchen Nr. 39, 43 und 46 geht hervor, daß bei einer Dosierung von je 10 Gew.-Teile/100 Gew.-Teile S-PVC das dreibasische Bleidimethylolpropionat mit den herkömmlichen Bleisalzen in den elektrischen Eigenschaften vergleichbar ist. So beträgt der spezifische Durchgangswiderstand der Probekörper stabilisiert mit zweibasischem Bleiphthalat $1{,}8 \times 10^{12}$ Ohm x cm, stabilisiert mit vierbasischem Bleifumarat $2{,}8 \times 10^{12}$ Ohm x cm und stabilisiert mit dreibasichem Bleidimethylolpropionat $2{,}7 \times 10^{12}$ Ohm x cm.

Des weiteren wurden die elektrischen Eigenschaften des dreibasischen Bleidimethylolpropionats in einer füllstoffhaltigen Kabelrezeptur untersucht.

In eine Mischung für Kabelmassen der Zusammensetzung

100 Gew.-Teile Suspensions-PVC, K-Wert 70
50 Gew.-Teile Di-2-äthylhexylphthalat
50 Gew.-Teile Calciumcarbonat
0,5 Gew.-Teile n-Paraffin
0,5 Gew.-Teile Wachsester
0,3 Gew.-Teile Bisphenol A

wurden jeweils 2,5 und 5,0 Gew.-Teile folgender basischer Bleisalze eingearbeitet: dreibasisches Bleisulfat, 0,5-basisches Bleicarbonat, zweibasisches Bleiphthalat, vierbasisches Bleifumarat, dreibasisches Bleidimethylolpropionat.

Die Mischungen wurden, wie für die Versuche Nr. 39 bis 46 beschrieben, entsprechend weiterverarbeitet und der spezifische Durchgangswiderstand nach DIN 53 482, VDE 0303, bei einer Temperatur von 23 °C und der rel. Luftfeuchte von 64 % gemessen.

Der Tabelle 5 mit den Versuchs-Beispielen 47 bis 56 ist zu entnehmen, daß mit dreibasischem Bleidimethylolpropionat stabilisierte Probekörper mit Probekörpern mit herkömmlichen Bleisalzen in den elektrischen Eigenschaften vergleichbar sind.

Beispiel zur Herstellung von dreibasischem Bleidimethylolpropionat

In einem zylindrischen heizbaren Reaktionsgefäß mit 2 l Inhalt, ausgestattet mit einem Propellerrührwerk, werden 1200 g Wasser vorgelegt und unter Rühren bei Raumtemperatur 49,7 g ( = 0,37 mol ) Dimethylolpropionsäure ( 99,8 %-ig) eingetragen. Anschließend wird der Ansatz auf 80 °C aufgeheizt. Bei dieser Temperatur wird die Säurelösung mit - je nach Reaktivität der Bleiglätte - 0,1 bis 1,1 g Ameisensäure ( 85 %-ig ) versetzt. Dann werden 165,2 g Bleiglätte ( = 0,74 mol ) innerhalb von 30 Minuten eingerührt. Nach beendeter Bleiglättezugabe ist der Ansatz viskos und von hellgelber Farbe. Sodann wird der Ansatz noch 4 Stunden lang bei einer Temperatur von 80 °C gerührt, wonach die Suspension leicht viskos und weißfarben ist. Die Suspension wird anschließend 48 Std. bei 105 °C getrocknet. Anschließend wird das getrocknete Bleisalz in einem Multimix-Gerät gemahlen. Der Bleigehalt des so erhaltenen weißfarbenen dreibasischen Bleidimethylolpropionats wurde zu 73,3 % ermittelt. Die Restfeuchte beträgt 0,2 %. Die Dichte des neuen Bleisalzes ist 4,5 $g/cm^3$.

Die Oberflächenmorphologie des dreibasischen Bleidimethylolpropionats ist in den Abbildungen der anliegenden REM-Aufnahmen dargestellt.

Tabelle 1 a Stabilisierung von Weich-PVC-Massen mit basischen Bleisalzen ( Versuche 1 bis 10 )

| Versuch Nr. | 1 | 2 | 3 | 4 | 5 | 6 | 7 | 8 | 9 | 10 |
|---|---|---|---|---|---|---|---|---|---|---|
| Suspensions-PVC, K-Wert 70 | 100 | 100 | 100 | 100 | 100 | 100 | 100 | 100 | 100 | 100 |
| Di-2-äthylhexyl-phthalat | 50 | 50 | 50 | 50 | 50 | 50 | 50 | 50 | 50 | 50 |
| n-Paraffin | 0,5 | 0,5 | 0,5 | 0,5 | 0,5 | 0,5 | 0,5 | 0,5 | 0,5 | 0,5 |
| E-Wachs (Wachsester) | 0,5 | 0,5 | 0,5 | 0,5 | 0,5 | 0,5 | 0,5 | 0,5 | 0,5 | 0,5 |
| Bisphenol A | 0,3 | 0,3 | 0,3 | 0,3 | 0,3 | 0,3 | 0,3 | 0,3 | 0,3 | 0,3 |
| Dreibasisches Bleisulfat | 1 | | | | | 2,5 | | | | |
| 0,5-basisches Bleicarbonat (Bleiweiß) | | 1 | | | | | 2,5 | | | |
| Zweibasisches Bleiphthalat | | | 1 | | | | | 2,5 | | |
| Vierbasisches Bleifumarat | | | | 1 | | | | | 2,5 | |
| Dreibasisches Bleidimethylol-propionat | | | | | 1 | | | | | 2,5 |
| HCl-Wert, min (VDE 0271) | 27 | 25 | 28 | 26 | 34 | 74 | 69 | 67 | 79 | 92 |

Tabelle 1 b    Stabilisierung von Weich-PVC-Massen mit basischen Bleisalzen ( Versuche 11 bis 20 )

| Versuch Nr. | 11 | 12 | 13 | 14 | 15 | 16 | 17 | 18 | 19 | 20 |
|---|---|---|---|---|---|---|---|---|---|---|
| Suspensions-PVC, K-Wert 70 | 100 | 100 | 100 | 100 | 100 | 100 | 100 | 100 | 100 | 100 |
| Di-2-äthylhexyl-phthalat | 50 | 50 | 50 | 50 | 50 | 50 | 50 | 50 | 50 | 50 |
| n-Paraffin | 0,5 | 0,5 | 0,5 | 0,5 | 0,5 | 0,5 | 0,5 | 0,5 | 0,5 | 0,5 |
| E-Wachs (Wachsester) | 0,5 | 0,5 | 0,5 | 0,5 | 0,5 | 0,5 | 0,5 | 0,5 | 0,5 | 0,5 |
| Bisphenol A | 0,3 | 0,3 | 0,3 | 0,3 | 0,3 | 0,3 | 0,3 | 0,3 | 0,3 | 0,3 |
| Dreibasisches Bleisulfat | 3,8 | | | | | 5 | | | | |
| 0,5-basisches Bleicarbonat (Bleiweiß) | | 3,8 | | | | | 5 | | | |
| Zweibasisches Bleiphthalat | | | 3,8 | | | | | 5 | | |
| Vierbasisches Bleifumarat | | | | 3,8 | | | | | 5 | |
| Dreibasisches Bleidimethylol-propionat | | | | | 3,8 | | | | | 5 |
| HCl-Wert, min (VDE 0271) | 123 | 130 | 137 | 154 | 152 | 177 | 249 | 165 | 227 | 253 |

Tabelle 2 Synergistische Wirkung des dreibasischen Bleidimethylolpropionats mit herkömmlichen Bleistabilisatoren bei der Stabilisierung von Weich-PVC-Massen

( Versuche 21 bis 28 )

| Versuch Nr. | 21 | 22 | 23 | 24 | 25 | 26 | 27 | 28 |
|---|---|---|---|---|---|---|---|---|
| Suspensions-PVC, K-Wert 70 | 100 | 100 | 100 | 100 | 100 | 100 | 100 | 100 |
| Di-2-äthylhexyl-phthalat | 50 | 50 | 50 | 50 | 50 | 50 | 50 | 50 |
| n-Paraffin | 0,5 | 0,5 | 0,5 | 0,5 | 0,5 | 0,5 | 0,5 | 0,5 |
| Wachsester | 0,5 | 0,5 | 0,5 | 0,5 | 0,5 | 0,5 | 0,5 | 0,5 |
| Bisphenol A | 0,3 | 0,3 | 0,3 | 0,3 | 0,3 | 0,3 | 0,3 | 0,3 |
| Dreibasisches Bleisulfat | 1,25 | | | | 2,5 | | | |
| 0,5-basisches Bleicarbonat (Bleiweiß) | | 1,25 | | | | 2,5 | | |
| Zweibasisches Bleiphthalat | | | 1,25 | | | | 2,5 | |
| Vierbasisches Bleifumarat | | | | 1,25 | | | | 2,5 |
| Dreibasisches Bleidimethylol-propionat | 1,25 | 1,25 | 1,25 | 1,25 | 2,5 | 2,5 | 2,5 | 2,5 |
| HCl-Wert, min (VDE 0271) | 93 | 99 | 102 | 98 | 263 | 340 | 254 | 313 |

Tabelle 3 Stabilisierung von Weich-PVC-Massen mit basischen Bleistabilisatoren

( Versuche 29 bis 38 )

| Versuch Nr. | 29 | 30 | 31 | 32 | 33 | 34 | 35 | 36 | 37 | 38 |
|---|---|---|---|---|---|---|---|---|---|---|
| Suspensions-PVC, K-Wert 70 | 100 | 100 | 100 | 100 | 100 | 100 | 100 | 100 | 100 | 100 |
| Di-iso-decyl-phthalat | 50 | 50 | 50 | 50 | 50 | 50 | 50 | 50 | 50 | 50 |
| n-Paraffin | 0,5 | 0,5 | 0,5 | 0,5 | 0,5 | 0,5 | 0,5 | 0,5 | 0,5 | 0,5 |
| E-Wachs (Wachsester) | 0,5 | 0,5 | 0,5 | 0,5 | 0,5 | 0,5 | 0,5 | 0,5 | 0,5 | 0,5 |
| Bisphenol A | 0,3 | 0,3 | 0,3 | 0,3 | 0,3 | 0,3 | 0,3 | 0,3 | 0,3 | 0,3 |
| Dreibasisches Bleisulfat | 2,5 | | | | | 5,0 | | | | |
| 0,5-basisches Bleicarbonat (Bleiweiß) | | 2,5 | | | | | 5,0 | | | |
| Zweibasisches Bleiphthalat | | | 2,5 | | | | | 5,0 | | |
| Vierbasisches Bleifumarat | | | | 2,5 | | | | | 5,0 | |
| Dreibasisches Bleidimethylol-propionat | | | | | 2,5 | | | | | 5,0 |
| HCl-Wert bei 200°C (VDE 0271) min | 74 | 63 | 61 | 69 | 94 | 134 | 179 | 158 | 198 | 233 |

Tabelle 4 Stabilisierung von Weich-PVC-Massen mit basischen Bleisalzen ( Versuche 39 bis 46 )

| Versuch Nr. | 39 | 40 | 41 | 42 | 43 | 44 | 45 | 46 |
|---|---|---|---|---|---|---|---|---|
| Suspensions-PVC, K-Wert 70 | 100 | 100 | 100 | 100 | 100 | 100 | 100 | 100 |
| Tri-n-$C_8$-$C_{10}$-alkyl-trimellitat | 50 | 50 | 50 | 50 | 50 | 50 | 50 | 50 |
| n-Paraffin | 0,5 | 0,5 | 0,5 | 0,5 | 0,5 | 0,5 | 0,5 | 0,5 |
| E-Wachs (Wachsester) | 1,0 | 1,0 | 1,0 | 1,0 | 1,0 | 1,0 | 1,0 | 1,0 |
| Bisphenol A | 0,25 | 0,25 | 0,25 | 0,25 | 0,25 | 0,25 | 0,25 | 0,25 |
| Zweibasisches Bleiphthalat | 10 | 12 | 13 | | | | | |
| Vierbasisches Bleifumarat | | | | 8 | 10 | | | |
| Dreibasisches Bleidimethylol-propionat | | | | | | 7 | 8 | 10 |
| HCl-Wert bei 200°C (VDE 0271) min | 355 | 397 | 418 | 368 | 432 | 405 | 464 | 570 |
| HCl-Wert bei 200°C (VDE 0271) nach 14 Tage Lagerung bei 140°C, min | 75 | 120 | 143 | 123 | 171 | 122 | 166 | 240 |
| Spezifischer Durchgangs-widerstand bei 23°C (DIN 53482, VDE 0303) in $10^{12}$ Ohm x cm | 1,8 | 2,4 | 2,5 | 2,7 | 2,8 | 2,5 | 2,6 | 2,7 |

Tabelle 5 Elektrische Eigenschaften von Weich-PVC-Massen mit basischen Bleisalzen

( Versuche 47 bis 56 )

| Versuch Nr. | 47 | 48 | 49 | 50 | 51 | 52 | 53 | 54 | 55 | 56 |
|---|---|---|---|---|---|---|---|---|---|---|
| Suspensions-PVC, K-Wert 70 | 100 | 100 | 100 | 100 | 100 | 100 | 100 | 100 | 100 | 100 |
| Di-2-äthylhexyl-phthalat | 50 | 50 | 50 | 50 | 50 | 50 | 50 | 50 | 50 | 50 |
| Calciumcarbonat | 50 | 50 | 50 | 50 | 50 | 50 | 50 | 50 | 50 | 50 |
| n-Paraffin | 0,5 | 0,5 | 0,5 | 0,5 | 0,5 | 0,5 | 0,5 | 0,5 | 0,5 | 0,5 |
| E-Wachs | 0,5 | 0,5 | 0,5 | 0,5 | 0,5 | 0,5 | 0,5 | 0,5 | 0,5 | 0,5 |
| Bisphenol A | 0,3 | 0,3 | 0,3 | 0,3 | 0,3 | 0,3 | 0,3 | 0,3 | 0,3 | 0,3 |
| Dreibasisches Bleisulfat | 2,5 | | | | | 5,0 | | | | |
| 0,5-basisches Bleicarbonat (Bleiweiß) | | 2,5 | | | | | 5,0 | | | |
| Zweibasisches Bleiphthalat | | | 2,5 | | | | | 5,0 | | |
| Vierbasisches Bleifumarat | | | | 2,5 | | | | | 5,0 | |
| Dreibasisches Bleidimethylol-propionat | | | | | 2,5 | | | | | 5,0 |
| Spezifischer Durchgangswiderstand bei 23°C (DIN 53482, VDE 0303) in $10^{13}$ Ohm x cm | 2,8 | 1,8 | 1,8 | 2,5 | 2,6 | 2,6 | 2,5 | 3,5 | 4,0 | 3,9 |

**Ansprüche**

1. Dreibasisches Bleidi-[2-2-Bis(hydroxymethyl)propionat] (3-bas. Bleidimethylolpropionat) der Formel

$3PbO \times Pb\,[CH_3\text{-}C(CH_2OH)_2COO]_2$

2. Stabilisatorzusammensetzung für halogenhaltige Vinylpolymerisate, insbesondere weichmacherhaltige, halogenhaltige Vinylpolymerisate, auf Basis basischer Bleisalze sowie ggf. mit weiteren üblichen Stabilisatoren, Hilfs- und Füllstoffen, dadurch gekennzeichnet, daß sie dreibasisches Bleidimethylolpropionat und ein Antioxydans enthält.

3. Stabilisatorzusammensetzung nach Anspruch 1, dadurch gekennzeichnet, daß sie ein Antioxydans aus der Gruppe Bisphenol A, $\alpha$-Phenylindol und Kondensationsprodukt aus 3-Methyl-6-tert.butylphenol und Crotonaldehyd enthält.

4. Stabilisatorzusammensetzung nach den Ansprüchen 2 und 3, dadurch gekennzeichnet, daß sie 5 bis 99,5 Gew.-% 3-bas. Bleidimethylolpropionat, 0 bis 87 Gew.-% Bleistabilisatoren aus der Gruppe neutrales oder basisches Bleistearat, Bleiweiß, 3-bas. oder 4-bas. Bleisulfat, 2-bas. Bleiphosphit, 2-bas. Bleisulfit, 2-bas. Bleiphthalat, 4-bas. Bleifumarat, 0,5 bis 8 Gew.-% Antioxidantien enthält.

METALLGESELLSCHAFT A.G., METALL-LABORATORIUM

RASTERELEKTRONENMIKROSKOPISCHE AUFNAHMEN

THEMA: Oberflächenmorphologie von dreibasischem Bleidimethylolpropionat

dreibasisches Bleidimethylolpropionat

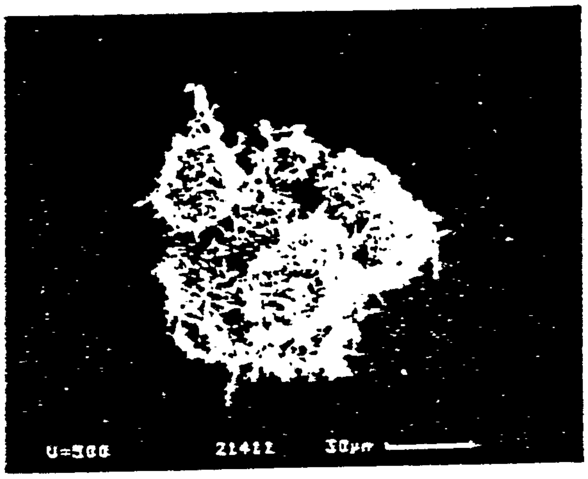


a)

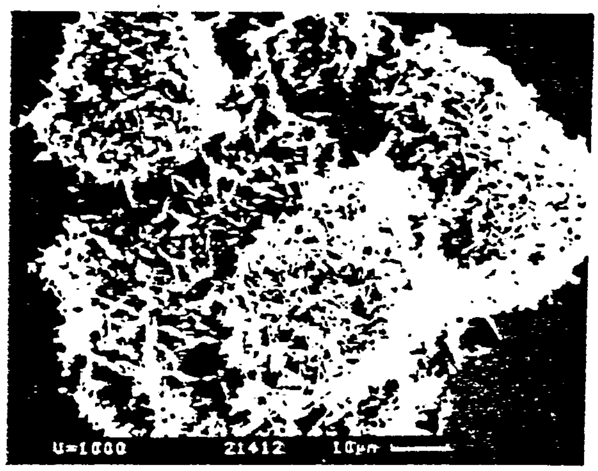


b)

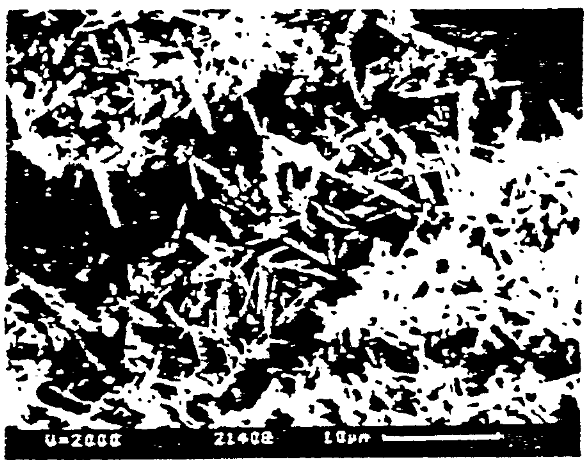


c)

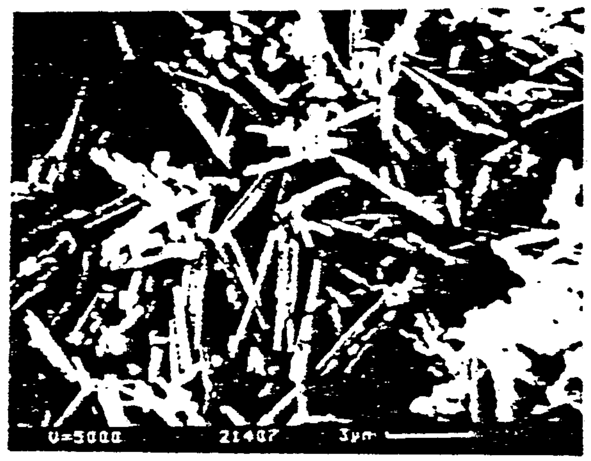


d)

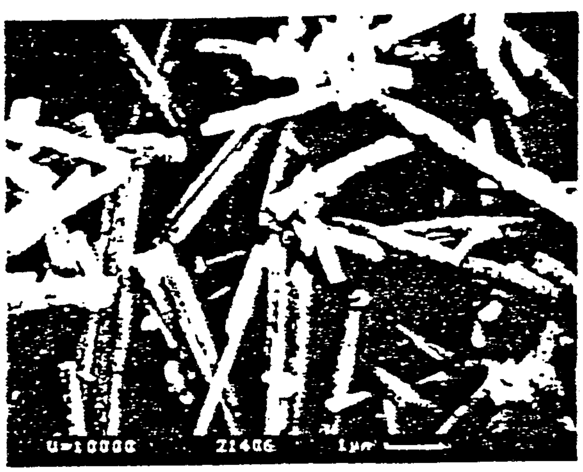


e)

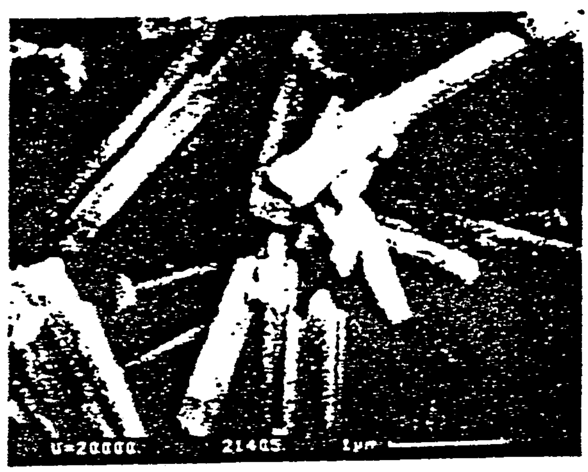


f)

Europäisches Patentamt

# EUROPÄISCHER RECHERCHENBERICHT

Nummer der Anmeldung

EP 88 20 1716

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-2 160 043 (BLEIBERGER BERGSWERKS-UNION)<br>* Ansprüche; Seite 4, Zeile 28 - Seite 5, Zeile 3 *<br>--- | 1,2 | C 07 C 59/10<br>C 08 K 5/00<br>C 08 L 57/08 //<br>(C 08 K 5/00<br>C 08 K 5:09<br>C 08 K 5:13 )<br>(C 08 K 5/00<br>C 08 K 5:09<br>C 08 K 5:34 )<br>(C 08 L 57/08<br>C 08 L 61:04 ) |
| A | FR-A-2 106 130 (LUTERSTAB LTD)<br>* Ansprüche *<br>----- | 1,2 | |
| | | | **RECHERCHIERTE SACHGEBIETE (Int. Cl.4)** |
| | | | C 07 C<br>C 08 K<br>C 08 L |

Der vorliegende Recherchenbericht wurde für alle Patentansprüche erstellt

| Recherchenort | Abschlußdatum der Recherche | Prüfer |
|---|---|---|
| DEN HAAG | 25-01-1989 | DE LOS ARCOS Y VELAZQUEZ |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)